(19) Europäisches Patentamt
European Patent Office
Office européen des brevets

(11) Veröffentlichungsnummer: **0 407 854 B1**

# (12) EUROPÄISCHE PATENTSCHRIFT

(45) Veröffentlichungstag der Patentschrift: **15.06.94**

(21) Anmeldenummer: **90112630.0**

(22) Anmeldetag: **03.07.90**

Die Akte enthält technische Angaben, die nach dem Eingang der Anmeldung eingereicht wurden und die nicht in dieser Patentschrift enthalten sind.

(51) Int. Cl.⁵: **C07D 249/08**, C07D 233/56, C07D 401/06, C07D 409/06, C07D 405/06, C07D 413/06, A01N 43/50, A01N 43/653

## (54) 1-Halogen-Vinylazole und diese enthaltende Fungizide.

(30) Priorität: **13.07.89 DE 3923153**

(43) Veröffentlichungstag der Anmeldung: **16.01.91 Patentblatt 91/03**

(45) Bekanntmachung des Hinweises auf die Patenterteilung: **15.06.94 Patentblatt 94/24**

(84) Benannte Vertragsstaaten: **AT BE CH DE DK ES FR GB GR IT LI NL SE**

(56) Entgegenhaltungen:
**EP-A- 0 004 315**
**EP-A- 0 047 057**
**EP-A- 0 060 223**
**EP-A- 0 076 628**
**EP-A- 0 227 100**

(73) Patentinhaber: **BASF Aktiengesellschaft**
**Carl-Bosch-Strasse 38**
**D-67063 Ludwigshafen(DE)**

(72) Erfinder: **Seele, Rainer, Dr.**
**Leiblstrasse 3**
**D-6701 Fussgoenheim(DE)**
Erfinder: **Kober, Reiner, Dr.**
**Im Schlittweg 20**
**D-6701 Fussgoenheim(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 407 854 B1

Rank Xerox (UK) Business Services
(3.10/3.09/3.3.3)

**Beschreibung**

Die vorliegende Erfindung betrifft neue Azolverbindungen, Verfahren zu ihrer Herstellung und diese enthaltende Fungizide.

Es ist bekannt, 1-(1,2,4-Triazol-1-yl)-2-(2-chlorphenyl)-2-(4-fluorphenyl)-ethen (EP 47057) als Fungizid zu verwenden. Die fungiziden Wirkungen sind jedoch nicht in allen Fällen befriedigend.

Es wurde nun gefunden, daß 1-Halogen-Vinylazole der allgemeinen Formel I

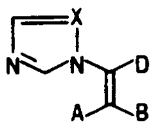

I

in welcher
A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,
D den Rest Chlor oder Brom bedeutet,
X den Rest CH oder N bedeutet,
sowie deren für Pflanzen verträgliche Säureadditionssalze oder Metallkomplexe, außer den Verbindungen, in denen D den Rest Chlor bedeutet und A und B identisch sind und 2-Chlorphenyl, 4-Chlorphenyl, 2,4-Dichlorphenyl, 3,4-Dichlorphenyl, 2-Methyl-4-chlorphenyl, 3-Methyl-4-chlorphenyl, 3-Trifluormethylphenyl, 3-Trifluormethyl-4-chlorphenyl, 4-Bromphenyl oder 2-Ethyl-4-chlorphenylbedeuten oder A 4-Chlorphenyl und B 2,4-Dichlorphenyl oder A 2-Chlorphenyl und B 4-Chlorphenyl oder A Phenyl und B 2,4-Dichlorphenyl bedeuten und X N bedeutet oder A und B identisch sind und 4-Chlorphenyl bedeuten und X CH bedeutet, eine bessere fungizide Wirkung besitzen als bekannte Azolverbindungen.

Die Verbindungen der Formel I enthalten eine mehrfach substituierte Doppelbindung und können daher als E/Z-Isomere auftreten. Die Gemische von E/Z-Isomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren.

Als fungizide Wirkstoffe können sowohl die einzelnen Diastereomere als auch deren Gemische verwendet werden.

A und B sind gleich oder verschieden und bedeuten beispielsweise 1-Naphthyl, 2-Naphthyl, p-Biphenyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, $C_1$-$C_4$-Alkoxyphenyl, 2-Methoxyphenyl, 3-Methoxyphenyl, 4-Methoxyphenyl, 2,4-Dimethoxyphenyl, $C_1$-$C_4$-Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, Halogen-$C_1$-$C_4$-alkylphenyl, 2-Trifluormethylphenyl, 3-Trifluoromethylphenyl, 4-Trifluormethylphenyl, Pyridyl, 3-Pyridyl, Furyl, 2-Furyl, Thienyl, 2-Thienyl, 3-Thienyl, Isoxazolyl, 5-Isoxazolyl.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i.a. nicht ankommt. Die Wirkstoffsalze werden hergestellt durch Umsetzung der 1-Halogen-Vinylazole (I) mit den Säuren.

Metallkomplexe der Wirkstoffe I oder ihrer Salze können z.B. mit Kupfer, Zink, Zinn, Mangan, Eisen, Kobalt oder Nickel gebildet werden, indem man die 1-Halogen-Vinylazole mit den Metallsalzen umsetzt.

Die Verbindungen der Formel I können hergestellt werden, indem man eine Verbindung der Formel II

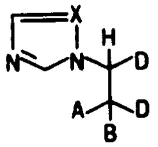

II

in der A, B, D und X die oben angegebene Bedeutung haben, mit Basen zur Umsetzung gebracht werden.

Die Reaktion erfolgt gegebenenfalls in Gegenwart eines Lösungs- oder Verdünnungsmittels unter Zusatz einer anorganischen oder organischen Base bei Temperaturen zwischen 10 und 120 °C.

Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ether wie Tetrahydrofuran, Diethylether, Dimethoxyethan, Dioxan oder Diisopropylether, Amide wie Dimethylformamid, Dimethylacetamid oder N-Methylpyrrolidon, Sulfolan oder entsprechende Gemische.

Die Umsetzung wird im allgemeinen bei Temperaturen zwischen 20 und 150 °C, drucklos oder unter Druck, kontinuierlich oder diskontinuierlich durchgeführt.

Die Ausgangsverbindungen II können beispielsweise hergestellt werden, indem man eine Verbindung der Formel III

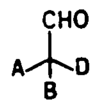

III

in welcher A, B und D die oben angegebenen Bedeutungen haben, mit einer Verbindung der Formel IV

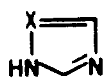

IV

in welcher X die angegebene Bedeutung hat, in Gegenwart von Thionylhalogeniden ($SOD_2$) zur Umsetzung bringt.

Die Umsetzung erfolgt gegebenenfalls in Gegenwart eines Lösungs- oder Verdünnungsmittels bei Temperaturen zwischen -30 und 80 °C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die Verbindungen der Formel II können weiterhin hergestellt werden, indem man eine Verbindung der Formel V

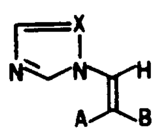

V

in welcher A, B und X die oben angegebene Bedeutung haben mit Chlor oder Brom in Gegenwart von Lewissäuren wie z.B. Zinkchlorid oder Zinkbromid umsetzt.

Die Reaktion erfolgt gegebenenfalls in Gegenwart eines Lösungs- oder Verdünnungsmittels bei Temperaturen zwischen -30 und 100 °C. Zu den bevorzugten Lösungsmitteln gehören Ester wie Essigsäureethylester, Essigsäuremethylester oder Essigsäurebutylester, Ether wie Tetrahydrofuran, Diethylether, Dimethoxyethan, Dioxan oder Diisopropylether und insbesondere Kohlenwasserstoffe und Chlorkohlenstoffe wie Pentan, Hexan, Toluol, Methylenchlorid, Chloroform, Tetrachlorkohlenstoff, Dichlorethan oder entsprechende Gemische.

Als Lewissäuren kommen vorzugsweise Metallhalogenide wie Zinkchlorid, Zinkbromid, Zinnchlorid, Zinnbromid, Eisentribromid, Aluminiumtrichlorid und Titantetrachlorid in Frage.

Die Verbindungen der Formel V können nach bekannten Verfahren (siehe EP 60 223, EP 47 057) hergestellt werden.

Die folgenden Beispiele erläutern die Herstellung der Wirkstoffe.

I. Herstellung der Ausgangsstoffe

Vorschrift 1

1,2-Dichlor-1-(1,2,4-triazol-1-yl)-2-(2-fluorphenyl)-2-(4-fluor-phenyl)-ethan

Zu einer Lösung von 22,4 g 1-(1,2,4-Triazol-1-yl)-2-(2-fluorphenyl)-2-(4-fluorphenyl)-ethen in 80 ml Tetrachlorkohlenstoff werden 1,1 g Zinkchlorid gegeben und anschließend 8,4 g Chlor eingegast. Nachdem das Reaktionsgemisch zwei Stunden bei Raumtemperatur (20 °C) gerührt hatte, wird der entstandene Niederschlag abgesaugt, in Methylenchlorid aufgenommen, mehrmals mit gesättigter Natriumhydrogencarbonat-Lösung gewaschen, über Natriumsulfat getrocknet und eingeengt. Man erhält 17,8 g (63 %) 1,2-Dichlor-1-(1,2,4-triazol-1-yl)-2-(2-fluorphenyl)-2-(4-fluor-phenyl)-ethan als 1:1-Diastereomerengemisch.

II. Herstellung der Endprodukte

Herstellungsbeispiel 1

1-Chlor-1-(1,2,4-triazol-1-yl)-2-(2-fluorphenyl)-2-(4-fluorphenyl)-ethen (Verbindung Nr. 1)

Zu einer Lösung von 17,8 g (0,0503 mol) 1,2-Dichlor-1-(1,2,4-triazol-1-yl)-2-(2-fluorphenyl)-2-(4-fluorphenyl)-ethan in 125 ml Methanol werden 5,4 g Natriummethylat gegeben. Nachdem das Reaktionsgemisch für eine Stunde unter Rückfluß erhitzt wurde, werden der Lösung 100 ml Wasser zugesetzt und mehrmals mit Methyl-tert.-butylether ausgeschüttelt. Die isolierte organische Phase wird zweimal mit Wasser gewaschen, über Natriumsulfat getrocknet und eingeengt, wobei 15,5 g (97 %) 1-Chlor-1-(1,2,4-triazol-1-yl)-2-(2-fluorphenyl)-2-(4-fluorphenyl)-ethan als 1:1-E/Z-Gemisch erhalten werden.

Entsprechend Beispiel 1 können die in der Tabelle aufgeführten Verbindungen hergestellt werden.

Tabelle

| Bsp | A | B | D | X | Schmp./IR | Isomer |
|---|---|---|---|---|---|---|
| 1 | 4-F-$C_6H_4$ | 2-F-$C_6H_4$ | Cl | N | 1603, 1508, 1276, 1233, 759 $cm^{-1}$ | E/Z=1:1 |
| 2 | 4-F-$C_6H_4$ | 2-F-$C_6H_4$ | Cl | CH | | |
| 3 | 4-F-$C_6H_4$ | 2-F-$C_6H_4$ | Br | N | Harz | E/Z=1:1 |
| 4 | 4-F-$C_6H_4$ | 2-F-$C_6H_4$ | Br | CH | | |
| 5 | 4-F-$C_6H_4$ | 3-F-$C_6H_4$ | Cl | N | | |
| 6 | 4-F-$C_6H_4$ | 4-F-$C_6H_4$ | Cl | N | 122-125°C | - |
| 7 | 4-F-$C_6H_4$ | 4-F-$C_6H_4$ | Cl | CH | | |
| 8 | 4-F-$C_6H_4$ | 4-F-$C_6H_4$ | Br | N | | |
| 9 | 4-F-$C_6H_4$ | 4-F-$C_6H_4$ | Br | CH | | |
| 10 | 4-F-$C_6H_4$ | $C_6H_5$ | Cl | N | 1604, 1507, 1270, 1231 1132, 846, 699 $cm^{-1}$ | E/Z=1:1 |
| 11 | 4-F-$C_6H_4$ | $C_6H_5$ | Br | N | | |
| 12 | 4-F-$C_6H_4$ | $C_6H_5$ | Cl | CH | | |
| 13 | 4-F-$C_6H_4$ | 2-Cl-$C_6H_4$ | Cl | N | 1602, 1507, 1276, 1234 753 $cm^{-1}$ | E/Z=1:1 |
| 14 | 4-F-$C_6H_4$ | 2-Cl-$C_6H_4$ | Cl | CH | | |
| 15 | 4-F-$C_6H_4$ | 2-Cl-$C_6H_4$ | Br | N | | |
| 16 | 4-F-$C_6H_4$ | 2-Cl-$C_6H_4$ | Br | CH | | |
| 17 | 4-F-$C_6H_4$ | 3-Cl-$C_6H_4$ | Cl | N | | |
| 18 | 4-F-$C_6H_4$ | 4-Cl-$C_6H_4$ | Cl | N | | |
| 19 | 4-F-$C_6H_4$ | 4-Cl-$C_6H_4$ | Br | N | | |
| 20 | 4-F-$C_6H_4$ | 4-Cl-$C_6H_4$ | Cl | CH | | |
| 21 | 4-F-$C_6H_4$ | 2-Br-$C_6H_4$ | Cl | N | | |
| 22 | 4-F-$C_6H_4$ | p-Biphenyl | Cl | N | | |
| 23 | 4-F-$C_6H_4$ | 2-Naphthyl | Cl | N | | |

Tabelle (Fortsetzung)

| Bsp | A | B | D | X | Schmp./IR | Isomer |
|---|---|---|---|---|---|---|
| 24 | 4-F-$C_6H_4$ | 1-Naphtyl | Cl | N | | |
| 25 | 4-F-$C_6H_4$ | 2-$CH_3$-$C_6H_4$ | Cl | N | | |
| 26 | 4-F-$C_6H_4$ | 2-$CH_3$-$C_6H_4$ | Br | N | | |
| 27 | 4-F-$C_6H_4$ | 2-$CH_3$-$C_6H_4$ | Cl | CH | | |
| 28 | 4-F-$C_6H_4$ | 4-$CH_3$-$C_6H_4$ | Cl | N | | |
| 29 | 4-F-$C_6H_4$ | 2,4-di-$CH_3$-$C_6H_3$ | Cl | N | | |
| 30 | 4-F-$C_6H_4$ | 2-$CF_3$-$C_6H_4$ | Cl | N | | |
| 31 | 4-F-$C_6H_4$ | 2-$CF_3$-$C_6H_4$ | Br | N | | |
| 32 | 4-F-$C_6H_4$ | 2-$CF_3$-$C_6H_4$ | Cl | CH | | |
| 33 | 4-F-$C_6H_4$ | 3-$CF_3$-$C_6H_4$ | Cl | N | | |
| 34 | 4-F-$C_6H_4$ | 4-$CF_3$-$C_6H_4$ | Cl | N | | |
| 35 | 4-F-$C_6H_4$ | 4-$CF_3$-$C_6H_4$ | Br | N | | |
| 36 | 4-F-$C_6H_4$ | 4-$CF_3$-$C_6H_4$ | Cl | CH | | |
| 37 | 4-F-$C_6H_4$ | 3-$NO_2$-$C_6H_4$ | Cl | N | | |
| 38 | 4-F-$C_6H_4$ | 3-$NH_2$-$C_6H_4$ | Cl | N | | |
| 39 | 4-F-$C_6H_4$ | 2-$OCH_3$-$C_6H_4$ | Cl | N | | |
| 40 | 4-F-$C_6H_4$ | 2-$OCH_3$-$C_6H_4$ | Br | N | | |
| 41 | 4-F-$C_6H_4$ | 2-$OCH_3$-$C_6H_4$ | Cl | CH | | |
| 42 | 4-F-$C_6H_4$ | 3-$OCH_3$-$C_6H_4$ | Cl | N | | |
| 43 | 4-F-$C_6H_4$ | 3-$OCH_3$-$C_6H_4$ | Br | N | | |
| 44 | 4-F-$C_6H_4$ | 4-$OCH_3$-$C_6H_4$ | Cl | N | | |
| 45 | 4-F-$C_6H_4$ | 4-$OCH_3$-$C_6H_4$ | Br | N | | |
| 46 | 4-F-$C_6H_4$ | 4-$OCH_3$-$C_6H_4$ | Cl | CH | | |
| 47 | 4-F-$C_6H_4$ | 2-Pyridyl | Cl | N | | |
| 48 | 4-F-$C_6H_4$ | 3-Pyridyl | Cl | N | | |

Tabelle (Fortsetzung)

| Bsp | A | B | D | X | Schmp./IR | Isomer |
|---|---|---|---|---|---|---|
| 49 | 4-F-$C_6H_4$ | 4-Pyridyl | Cl | N | | |
| 50 | 4-F-$C_6H_4$ | 2-Thienyl | Cl | N | | |
| 51 | 4-F-$C_6H_4$ | 3-Thienyl | Cl | N | | |
| 52 | 4-F-$C_6H_4$ | 2-Furyl | Cl | N | | |
| 53 | 4-F-$C_6H_4$ | 5-Isoxazolyl | Cl | N | | |
| 54 | $C_6H_5$ | 2-F-$C_6H_4$ | Cl | N | | |
| 55 | $C_6H_5$ | $C_6H_5$ | Cl | N | | |
| 56 | $C_6H_5$ | 2-Cl-$C_6H_4$ | Cl | N | 1502, 1402, 1275, 1132, 857, 756, 699 $cm^{-1}$ | E/Z = 1:1 |
| 57 | $C_6H_5$ | 4-Cl-$C_6H_4$ | Cl | N | Harz | E/Z = 1:1 |
| 58 | $C_6H_5$ | 2-Br-$C_6H_4$ | Cl | N | | |
| 59 | $C_6H_5$ | 4-Br-$C_6H_4$ | Cl | N | | |
| 60 | $C_6H_5$ | 2-$CH_3$-$C_6H_4$ | Cl | N | | |
| 61 | $C_6H_5$ | 4-$CH_3$-$C_6H_4$ | Cl | N | | |
| 62 | $C_6H_5$ | 4-tert.-$C_4H_9$-$C_6H_4$ | Cl | N | | |
| 63 | $C_6H_5$ | 2-$CF_3$-$C_6H_4$ | Cl | N | | |
| 64 | $C_6H_5$ | 4-$CF_3$-$C_6H_4$ | Cl | N | | |
| 65 | $C_6H_5$ | 2-$OCH_3$-$C_6H_4$ | Cl | N | | |
| 66 | $C_6H_5$ | 4-$OCH_3$-$C_6H_4$ | Cl | N | | |
| 67 | $C_6H_5$ | 2-Naphthyl | Cl | N | | |
| 68 | $C_6H_5$ | 2-Pyridyl | Cl | N | | |
| 69 | $C_6H_5$ | 3-Pyridyl | Cl | N | | |
| 70 | $C_6H_5$ | 2-Thienyl | Cl | N | | |
| 71 | $C_6H_5$ | 3-Thienyl | Cl | N | | |
| 72 | 4-Cl-$C_6H_4$ | 2-F-$C_6H_4$ | Cl | N | | |

Tabelle (Fortsetzung)

| Bsp | A | B | D | X | Schmp./IR | Isomer |
|---|---|---|---|---|---|---|
| 73 | $4-Cl-C_6H_4$ | $2-Br-C_6H_4$ | Cl | N | | |
| 74 | $4-Cl-C_6H_4$ | $4-Br-C_6H_4$ | Cl | N | | |
| 75 | $4-Cl-C_6H_4$ | $2-CH_3-C_6H_4$ | Cl | N | | |
| 76 | $4-Cl-C_6H_4$ | $4-CH_3-C_6H_4$ | Cl | N | | |
| 77 | $4-Cl-C_6H_4$ | $2-CF_3-C_6H_4$ | Cl | N | | |
| 78 | $4-Cl-C_6H_4$ | $4-CF_3-C_6H_4$ | Cl | N | | |
| 79 | $4-Cl-C_6H_4$ | $2-OCH_3-C_6H_4$ | Cl | N | | |
| 80 | $4-Cl-C_6H_4$ | $4-OCH_3-C_6H_4$ | Cl | N | | |
| 81 | $4-Br-C_6H_4$ | $2-F-C_6H_4$ | Cl | N | | |
| 82 | $4-Br-C_6H_4$ | $2-Cl-C_6H_4$ | Cl | N | | |
| 83 | $4-Br-C_6H_4$ | $2-CH_3-C_6H_4$ | Cl | N | | |
| 84 | $4-Br-C_6H_4$ | $2-CF_3-C_6H_4$ | Cl | N | | |
| 85 | $4-Br-C_6H_4$ | $4-CF_3-C_6H_4$ | Cl | N | | |
| 86 | $4-Br-C_6H_4$ | $2-OCH_3-C_6H_4$ | Cl | N | | |
| 87 | $4-Br-C_6H_4$ | $4-OCH_3-C_6H_4$ | Cl | N | | |
| 88 | $4-CH_3-C_6H_4$ | $2-Cl-C_6H_4$ | Cl | N | | |
| 89 | $4-CH_3-C_6H_4$ | $2-F-C_6H_4$ | Cl | N | | |
| 90 | $4-CH_3-C_6H_4$ | $2-CH_3-C_6H_4$ | Cl | N | | |
| 91 | $4-CH_3-C_6H_4$ | $4-CH_3-C_6H_4$ | Cl | N | | |
| 92 | $4-CH_3-C_6H_4$ | $2-CF_3-C_6H_4$ | Cl | N | | |
| 93 | $4-CH_3-C_6H_4$ | $4-CF_3-C_6H_4$ | Cl | N | | |
| 94 | $4-CH_3-C_6H_4$ | $2-OCH_3-C_6H_4$ | Cl | N | | |
| 95 | $4-CH_3-C_6H_4$ | $4-OCH_3-C_6H_4$ | Cl | N | | |

Tabelle (Fortsetzung)

| Bsp | A | B | D | X | Schmp./IR | Isomer |
|---|---|---|---|---|---|---|
| 96 | $4-CF_3-C_6H_4$ | $2-Cl-C_6H_4$ | Cl | N | | |
| 97 | $4-CF_3-C_6H_4$ | $2-F-C_6H_4$ | Cl | N | | |
| 98 | $4-CF_3-C_6H_4$ | $2-CH_3-C_6H_4$ | Cl | N | | |
| 99 | $4-CF_3-C_6H_4$ | $2-CF_3-C_6H_4$ | Cl | N | | |
| 100 | $4-CF_3-C_6H_4$ | $2-OCH_3-C_6H_4$ | Cl | N | | |
| 101 | $4-OCH_3-C_6H_4$ | $2-Cl-C_6H_4$ | Cl | N | | |
| 102 | $4-OCH_3-C_6H_4$ | $2-F-C_6H_4$ | Cl | N | | |
| 103 | $4-OCH_3-C_6H_4$ | $2-CH_3-C_6H_4$ | Cl | N | | |
| 104 | $4-OCH_3-C_6H_4$ | $2-CF_3-C_6H_4$ | Cl | N | | |
| 105 | $4-OCH_3-C_6H_4$ | $2-OCH_3-C_6H_4$ | Cl | N | | |
| 106 | $4-OCH_3-C_6H_4$ | $4-OCH_3-C_6H_4$ | Cl | N | | |
| 107 | $2-F-C_6H_4$ | $2-Cl-C_6H_4$ | Cl | N | | |
| 108 | $2-F-C_6H_4$ | $2-F-C_6H_4$ | Cl | N | | |
| 109 | $2-F-C_6H_4$ | $2-CH_3-C_6H_4$ | Cl | N | | |
| 110 | $2-Cl-C_6H_4$ | $2-CH_3-C_6H_4$ | Cl | N | | |
| 111 | $2-Cl-C_6H_4$ | $2-CF_3-C_6H_4$ | Cl | N | | |
| 112 | $2-Cl-C_6H_4$ | $2-OCH_3-C_6H_4$ | Cl | N | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,

Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, insbesondere 0,01 bis 10 g, je kg Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 6 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 13 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 6 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 13 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 6 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 13 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 6 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 13 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 6 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Als Vergleichswirkstoff wurde 1-(1,2,4-Triazol-1-yl)-2-(2-chlorphenyl)-2-(4-fluorphenyl)-ethen (A) - bekannt aus EP 47 057 - benutzt.

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22 °C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22 °C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 6 und 13 bei der Anwendung als 0,025 %ige (Gew.%) Spritzbrühe eine bessere fungizide Wirkung zeigen (94 %) als der bekannte Vergleichswirkstoff A (55 %).

## Patentansprüche

**1.** 1-Halogen-Vinylazole der allgemeinen Formel I

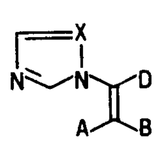

I

in welcher
A und B gleich oder verschieden sind und Phenyl, Biphenyl, Naphthyl oder Hetaryl bedeuten, wobei diese Reste einfach bis dreifach durch Halogen, Nitro, Phenoxy, Amino, Alkyl, Alkoxy oder Halogenalkyl mit jeweils 1- bis 4-C-Atomen substituiert sein können,
D den Rest Chlor oder Brom bedeutet,
X den Rest CH oder N bedeutet,
sowie deren für Pflanzen verträgliche Säureadditionssalze und Metallkomplexe, außer den Verbindungen, in denen D den Rest Chlor bedeutet und A und B identisch sind und 2-Chlorphenyl, 4-Chlorphenyl, 2,4-Dichlorphenyl, 3,4-Dichlorphenyl, 2-Methyl-4-chlorphenyl, 3-Methyl-4-chlorphenyl, 3-Trifluormethylphenyl, 3-Trifluormethyl-4-chlorphenyl, 4-Bromphenyl oder 2-Ethyl-4-chlorphenyl bedeuten oder A 4-Chlorphenyl und B 2,4-Dichlorphenyl oder A 2-Chlorphenyl und B 4-Chlorphenyl oder A Phenyl und B 2,4-Dichlorphenyl bedeuten und X N bedeutet oder A und B identisch sind und 4-Chlorphenyl bedeuten und X CH bedeutet.

**2.** Verfahren zur Herstellung der 1-Halogen-Vinylazole der Formel I gemäß Anspruch 1, dadurch gekennzeichnet, daß man eine Verbindung der Formel II

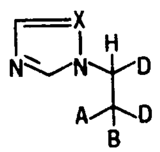

II

in welcher A, B, D und X die im Anspruch 1 angegebenen Bedeutungen haben, mit Basen zur Umsetzung bringt und die so erhaltenen Verbindungen gegebenenfalls in ihre für Pflanzen verträglichen Säureadditionssalze oder Metallkomplexe überführt.

**3.** Fungizides Mittel, enthaltend einen Trägerstoff und eine fungizid wirksame Menge eines 1-Halogen-Vinylazols der allgemeinen Formel I gemäß Anspruch 1.

**4.** Verfahren zur Bekämpfung von Pilzen, dadurch gekennzeichnet, daß man eine fungizid wirksame Menge eines 1-Halogen-Vinylazols der Formel I gemäß Anspruch 1 auf die Pilze oder durch Pilzbefall bedrohte Materialien, Flächen, Pflanzen oder Saatgüter einwirken läßt.

**5.** Verbindung der Formel I gemäß Anspruch 1, dadurch gekennzeichnet, daß X N, D Cl, A 4-Fluorphenyl und B 4-Fluorphenyl bedeuten.

**6.** Verbindung der Formel I gemäß Anspruch 1, dadurch gekennzeichnet, daß X N, D Cl, A 4-Fluorphenyl und B 2-Chlorphenyl bedeuten.

## Claims

**1.** A 1-halovinylazole of the general formula I

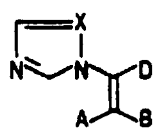

I

where
A and B are identical or different and are each phenyl, biphenyl, naphthyl or hetaryl, and these radicals may be monosubstituted to trisubstituted by halogen, nitro, phenoxy, amino, alkyl, alkoxy or haloalkyl, each of 1 to 4 carbon atoms,
D is chlorine or bromine and
X is CH or N,
or a plant-tolerated acid addition salt or metal complex thereof, except for the compounds in which D is chlorine and A and B are identical and are each 2-chlorophenyl, 4-chlorophenyl, 2,4-dichlorophenyl, 3,4-dichlorophenyl, 2-methyl-4-chlorophenyl, 3-methyl-4-chlorophenyl, 3-trifluoromethylphenyl, 3-trifluoromethyl-4-chlorophenyl, 4-bromophenyl or 2-ethyl-4-chlorophenyl or A is 4-chlorophenyl and B is 2,4-dichlorophenyl or A is 2-chlorophenyl and B is 4-chlorophenyl or A is phenyl and B is 2,4-dichlorophenyl and X is N or A and B are identical and are each 4-chlorophenyl and X is CH.

**2.** A process for the preparation of a 1-halovinylazole of the formula I as claimed in claim 1, which comprises reacting a compound of the formula II

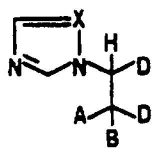

II

where
A, B, D and X have the meanings stated in claim 1, with a base and, if appropriate, converting the resulting compound into its plant-tolerated acid addition salt or metal complex.

**3.** A fungicide containing a carrier and a fungicidally effective amount of a 1-halovinylazole of the general formula I.

**4.** A method for controlling fungi, wherein a fungicidally effective amount of a 1-halovinylazole of the formula I as claimed in claim 1, is allowed to act on the fungi or the materials, areas, plants or seeds threatened by fungal attack.

**5.** A compound of the formula I as claimed in claim 1, wherein X is N, D is Cl, A is 4-fluorophenyl and B is 4-fluorophenyl.

**6.** A compound of the formula I as claimed in claim 1, wherein X is N, D is Cl, A is 4-fluorophenyl and B is 2-chlorophenyl.

**Revendications**

**1.** 1.halogène-vinylazoles de formule générale I

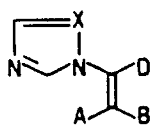

I.

dans laquelle
A et B sont identiques ou différents et représentent phényle, biphényle, naphtyle ou hétaryle, ces restes pouvant être substitués une à trois fois par halogène, nitro, phénoxy, amino, alkyle, alcoxy ou halogène-alkyle à 1 à 4 atomes C chacun,
D représente chlore ou brome,
X représente CH ou N,
ainsi que leurs sels d'addition d'acide et complexes métalliques tolérés par les plantes, exceptés les composés dans lesquels D représente chlore et A et B sont identiques et représentent 2-chlorophényle, 4-chlorophényle, 2,4-dichlorophényle, 3,4-dichlorophényle, 2-méthyl-4-chlorophényle, 3-méthyl-4-chlorophényle, 3-trifluorométhylphényle, 3-trifluorométhyl-4-chlorophényle, 4-bromophényle ou 2-éthyl-4-chlorophényle, ou A représente 4-chlorophényle et B 2,4-dichlorophényle ou A représente 2-chlorophényle et B 4-chlorophényle ou A représente phényle et B 2,4-dichlorophényle et X est mis pour N ou A et B sont identiques et représentent 4-chlorophényle et X est mis pour CH.

**2.** Procédé de préparation des 1-halogène-vinylazoles de formule I, selon la revendication 1, caractérisé par le fait que l'on met à réagir avec des bases un composé de formule II

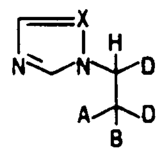

II

dans laquelle A, B, D et X ont les significations données dans la revendication 1, et on transforme les composés ainsi obtenus éventuellement en leurs sels d'addition d'acide ou complexes métalliques tolérés par les plantes.

**3.** Fongicide, contenant un support et une quantité efficace au point de vue fongicide d'un 1-halogène-vinylazole de formule générale I, selon la revendication 1.

**4.** Procédé de lutte contre les champignons, caractérisé par le fait que l'on fait agir une quantité efficace au point de vue fongicide d'un 1-halogène-vinylazolede formule I, selon la revendication 1, sur les champignons ou les matériaux, surfaces, plantes ou semences menacés par l'attaque par les champignons.

**5.** Composé de formule I, selon la revendication 1, caractérisa par le fait que X représente N, D représente Cl, A est mis pour 4-fluorophényle et B pour 4-fluorophényle.

**6.** Composé de formule I, selon la revendication 1, caractérisé par le fait que X représente N, D représente Cl, A est mis pour 4-fluorophényle et B pour 2-chlorophényle.